# EUROPÄISCHE PATENTSCHRIFT

(11) **EP 1 350 461 B1**
(45) Veröffentlichungstag und Bekanntmachung des Hinweises auf die Patenterteilung: **19.03.2008**
(21) Anmeldenummer: 03015712.7
(22) Anmeldetag: 23.12.1998
(51) Int. Cl.: A61B 5/022

(54) **Verfahren und Messgerät zur Bestimmung des Blutdrucks**
Method and measuring device for determining blood pressure
Procédé et appareil pour déterminer la pression sanguine

(30) Priorität: 24.12.1997 DE 19757974
(43) Veröffentlichungstag der Anmeldung: 08.10.2003
(62) Teilanmeldung aus: 98966668.0
(73) Patentinhaber: KAZ Europe SA, 1003 Lausanne (CH)
(72) Erfinder: Harttmann, Brigitte, 65527 Niedernhausen (DE); Giersiepen, Martin, Dr., 61440 Oberursel (DE); Kressmann, Frank, 65760 Eschborn (DE); Schnak, Fred, 61476 Kronberg (DE); Freund, Dirk, Dr., 65779 Kelkheim (DE)
(74) Vertreter: Grünecker, Kinkeldey, Stockmair & Schwanhäusser Anwaltssozietät

(56) Entgegenhaltungen:
- DE-U- 29 612 412
- US-A- 4 338 950
- US-A- 4 870 973
- PATENT ABSTRACTS OF JAPAN vol. 096, no. 012, 26. Dezember 1996 (1996-12-26) & JP 08 215161 A (OMRON CORP), 27. August 1996 (1996-08-27)
- PATENT ABSTRACTS OF JAPAN vol. 096, no. 012, 26. Dezember 1996 (1996-12-26) & JP 08 215162 A (OMRON CORP), 27. August 1996 (1996-08-27) & US 5 778 879 A 14. Juli 1998 (1998-07-14)
- PATENT ABSTRACTS OF JAPAN vol. 014, no. 024 (C-677), 18. Januar 1990 (1990-01-18) & JP 01 265939 A (MATSUSHITA ELECTRIC WORKS LTD), 24. Oktober 1989 (1989-10-24)
- PATENT ABSTRACTS OF JAPAN vol. 096, no. 012, 26. Dezember 1996 (1996-12-26) & JP 08 215160 A (OMRON CORP), 27. August 1996 (1996-08-27)

## Beschreibung

Die vorliegende Erfindung betrifft ein Verfahren gemäß dem Oberbegriff des Anspruchs 1. Die vorliegende Erfindung betrifft ferner ein Blutdruckmeßgerät gemäß dem Oberbegriff des Anspruchs 8.

Blutdruckmessungen am Handgelenk oder an einem Finger leiden oftmals an mangelnder Meßgenauigkeit und unzureichender Reproduzierbarkeit. Dies ist durch die hohe Empfindlichkeit der Messung bezüglich Schwankungen der Meßposition bedingt, d. h. der individuellen Lage des Handgelenkes bzw. des Fingers, relativ zur Lage des Herzens. Um exakte Ergebnisse zu erhalten, ist bei bekannten Meßgeräten eine Messung auf Herzhöhe erforderlich. Dies wird jedoch in der Regel nur näherungsweise eingehalten und wird von den Personen, bei denen die Blutdruckmessung durchgeführt wird, als beschränkend und unpraktisch empfunden. Den Messungen wohnt deshalb stets eine Ungenauigkeit inne. Im Falle einer von der Herzhöhe abweichenden Meßposition verfälscht die hydrostatische D!uckdifferenz das Meßergebnis um etwa 0,78 mm Hg/cm. Eine mangelhafte Lage während eines Meßvorganges führt somit zu einem systematischen Meßfehler. Zusätzlich kann eine kurzzeitige, eher zufällige Lageschwankung wie beispielsweise Zittern oder eine Armbewegung noch einen zweiten dynamischen Fehler, sog. Bewegungsartefakte, zur Folge haben, die die algorithmische Auswertung der eigentlichen Meßgröße erheblich erschweren, wenn nicht sogar unmöglich machen.

In der US-A 47 79 626 wurde bereits vorgeschlagen, bei einer Blutdruckmessung an einem Finger die hydrostatische Komponente des Blutdrucks mittels einer Vorrichtung zu kompensieren, die einen der hydrostatischen Druckkomponente entsprechenden Gegendruck bereitstellt. Hierzu wird auf Höhe des Herzens an der Brust ein Flüssigkeitsreservoir befestigt, das mittels eines Schlauches mit dem Blutdruckmeßgerät am Finger verbunden ist. Der Drucksensor des Meßgerätes ist dabei als Differenzdrucksensor ausgebildet, der die Druckdifferenz zwischen dem im Finger herrschenden Blutdruck und dem am Ende des Schlauches herrschenden Flüssigkeitsdruck mißt. Es liegt jedoch auf der Hand, daß dieses Blutdruckmeßgerät unhandlich und nur umständlich anzulegen ist. Darüber hinaus behindert der am Körper verlegte Schlauch die Bewegungsfreiheit der jeweiligen Person.

Die DE 296 12 412 U1 beschreibt ein Blutdruckmeßgerät, das an das Handgelenk anzulegen ist und bei dem im Gehäuse des Blutdruckmeßgerätes ein Pendel angeordnet ist. Die Außenseite des Pendels ist mit einer Farbskalierung versehen, so daß abhängig von der Armbeugenstellung eine bestimmte Farbe sichtbar wird. Hierbei ist die Hanbhabung u.a. insoweit erschwert, als daß das Pendel sich erst ausschwingen muß bis eine Ablesung möglich ist.

Ferner ist aus der JP 08 215162 A ein Blutdruckmessgerät bekannt, dass einen Sensor zur Detektierung der Herztöne des Benutzers aufweist, um so eine Messung auf Herzhöhe sicherzustellen.

Der vorliegenden Erfindung liegt daher die Aufgabe zugrunde, ein Verfahren und ein Gerät zur Bestimmung des Blutdruckes zu schaffen, das bei hoher Meßgenauigkeit und ausreichender Reproduzierbarkeit der Blutdruckbestimmung einfach zu handhaben ist.

In Verfahrenstechnischer Hinsicht wird diese Aufgabe durch ein Verfahren nach Anspruch 1 gelöst. Die Ausrichtungserfassungseinheit bzw. der Neigungssensor gibt ein elektrisches Signal je nach der detektierten Ausrichtung des Körpergliedes ab und dieses elektrische Signal wird weiter verarbeitet. Durch die elektrische Erfassung der Ausrichtung des Körpergliedes sind vielfältige Weiterverarbeitungsoptionen des elektrischen Signales möglich, die zu einer optimalen Handhabung und erhöhter Meßgenauigkeit der Blutdruckbestimmung führen. Das in der Ausrichtungserfassungseinheit vorgesehene bewegliche Teil, daß z.B. pendelartig ausgebildet ist, weist eine bestimmte Schwingeigenfrequenz auf, die eine Periodendauer der Schwingung festlegt. Abhängig von der Art der Bewegung des Patienten wird somit eine unterschiedlich starke Schwingungsamplitude in der Ausrichtungserfassungseinheit erzeugt, die zunächst maximal anschwingt und eine gewisse Zeit zum ausschwingen benötigt. Durch die elektrische Weiterverarbeitung des Meßsignales ist es somit möglich, dieses Signal zu integrieren über die Zeit bzw. einen Mittelwert zu bilden über die Periodendauer, so daß das Signal stark gedämpft und damit letztlich erst ablesbar oder weiter nutzbar wird, da kleinere Ausschläge und ein kürzeres Nachschwingen des beweglichen Mittels in der Ausrichtungserfassungseinheit erzeugt wird. Es wird somit ein Problem gelöst, daß vor allem bei einer sehr kompakten Ausrichtungserfassungseinheit, die innerhalb eines Blutdruckmeßgerätegehäuses angeordnet ist auftreten kann. Weitere Optionen zur Weiterverarbeitung des elektrischen Signals werden nachfolgend aufgeführt.

Der erfaßte Blutdruck in einer Auswerteeinheit kann entsprechend der erfaßten Ausrichtung des Körperglieds korrigiert werden. Bei einer vorgegebenen Ausrichtung des Körpers, insbesondere einer aufrechten Haltung des Oberkörpers, kann mit der Erfassungseinheit die absolute Ausrichtung des Körperglieds im Raum erfaßt und dadurch die Stellung des Körperglieds relativ zum Herzen bestimmt werden. Dementsprechend kann der tatsächlich gemessene Blutdruck im Körperglied in Abhängigkeit eines elektrischen Ausrichtungssignales der Erfassungseinheit korrigiert und der auf Herzhöhe herrschende Blutdruck bestimmt werden.

Gemäß einer bevorzugten Ausführung der Erfindung wird mit einem Neigungssensor die Winkelstellung des Körperglieds, insbesondere des Unterarmes, erfaßt. Der erfaßte Blutdruck kann entsprechend der Winkelstellung korrigiert werden. Insbesondere kann die Neigung des Unterarmes zur Horizontalen bzw. Vertikalen erfaßt werden, die bei einer vorgegebenen Position des Ellenbogens, beispielsweise in einer am Oberkörper anliegenden Stellung, ein Maß für die Höhenlage des Handgelenkes und damit der hydrostatischen Komponente des Blutdrucks im Handgelenk ist.

Um eine einfache Signalverarbeitung zu ermöglichen, wird vorzugsweise die Ausrichtung des Körperglieds und der Blutdruck in demselben nacheinander erfaßt. Dieser ermöglicht es, bei ausreichender Genauigkeit, das Ausrichtungs- und Drucksignal nacheinander zu verarbeiten.

Gemäß einer anderen bevorzugten Ausführung der Erfindung wird die Ausrichtung des Körperglieds zeitparallel zur Druckmessung erfaßt. Dies erlaubt eine erhöhte Genauigkeit der Messung, da der Korrektur des erfaßten Blutdrucks stets die jeweilige Stellung des Körperglieds zugrunde gelegt werden kann.

In vorteilhafter Weiterbildung der Erfindung kann während der Druckerfassung eine Bewegung, insbesondere eine Beschleunigung des Körperglieds, an dem der Blutdruck gemessen wird, erfaßt werden. Der erfaßte Blutdruck wird dann entsprechend der erfaßten Bewegung korrigiert. Hierdurch können kurzzeitige Lageänderungen, wie z. B. Zittern während der Messung erfaßt werden. Durch eine Rückrechnung von den gemessenen Lageschwankungen auf die dazu korrespondierenden Druckschwankungen kann der Einfluß von Bewegungsartefakten verringert werden. Vorzugsweise wird die Ausrichtung und die Bewegung des Körperglieds kontinuierlich erfaßt.

Hinsichtlich des Gerätes wird die obengenannte Aufgabe mit einem Blutdruckmeßgerät erfindungsgemäß mit den Merkmalen des Anspruchs 8 gelöst. Es ist durch die Ausrichtungserfassungseinheit zur Weiterverarbeitung ein elektrisches Signal in Abhängigkeit von der Ausrichtung des Körpersignals abgebbar. Somit können Schwingungen in der Ausrichtungserfassungseinheit auf ein genau spezifiziertes Dämpfungsverhalten eingestellt werden ohne den Nachteil eine aufwendige mechanische Dämpfung, die zu dem fertigungsabhängig in ihrer Genauigkeit ist, vornehmen zu müssen.

Bevorzugt weist die Auswerteeinheit eine Korrektureinheit zur Korrektur des Drucksignales entsprechend der erfaßten Ausrichtung auf. Die Ausrichtungs-Erfassungseinheit erzeugt ein elektrisches Signal, das der Ausrichtung entspricht und das in der Korrektureinheit zur Korrektur des Drucksignales verarbeitet wird.

Die Ausrichtungs-Erfassungseinheit weist in vorteilhafter Weise einen Neigungssensor auf, der die Neigung des Körperglieds, an dem der Drucksensor angelegt ist, bzw. die Neigung der Anlegeeinheit, die der Neigung des Körperglieds entspricht, erfaßt. Vorzugsweise ist der Neigungssensor derart ausgebildet, daß er die absolute Neigung des Körperglieds, d. h. den Winkel zur Horizontalen bzw. Vertikalen erfaßt. Der Neigungswinkel gibt ein Maß für die Stellung des Körperglieds an.

Gemäß einer bevorzugten Ausführung der Erfindung kann eine Bewegungs-Erfassungseinheit zur Erfassung einer Bewegung, insbesondere einer Beschleunigung des Körperglieds vorgesehen sein, wobei ein elektrisches Bewegungssignal in der Auswerteeinheit verarbeitet wird. Durch das Bewegungssignal ist das Maß an motorischer Aktivität der jeweiligen Person feststellbar. Auf diese Weise läßt sich beurteilen, ob sich der Anwender generell in einem Zustand ausreichender Ruhe befindet und eine Messung überhaupt sinnvoll durchführbar ist.

Dabei kann die Messung dann, wenn ein zu ungenaues Ergebnis geliefert werden würde, unterbunden werden oder zumindest ein Hinweis auf die geringe Aussagekraft der Messung auf einer Anzeigevorrichtung angezeigt werden. Vorteilhafterweise kann das erfaßte Drucksignal in Abhängigkeit des elektrischen Bewegungssignales in der Korrektureinheit der Auswerteeinheit korrigiert werden.

Vorzugsweise umfaßt die Bewegungs-Erfassungseinheit den Neigungssensor und eine mit diesem verbundene Differenziereinheit, die die zeitliche erste oder zweite Ableitung des Neigungssignales bildet und das errechnete Signal als Bewegungssignal bereitstellt.

Entsprechend einer bevorzugten Ausführung der Erfindung sind die Ausrichtungs-Erfassungseinheit und der Drucksensor mit der Auswerteeinheit über eine Zeitschalteinheit verbunden. Die Zeitschalteinheit zur zeitabhängigen Durchschaltung des jeweiligen Signales an die Auswerteeinheit kann als Schaltweiche bzw. Multiplexer ausgebildet sein.

Zwischen die Schalteinheit und die Auswerteeinheit ist zumindest ein Analog-Digital-Wandler geschaltet. Um die Ausrichtung des Körperglieds zeitparallel zur Druckmessung erfassen zu können, sind bevorzugt zwei Analog-Digital-Wandler vorgesehen.

Zweckmäßigerweise weist das Blutdruckmeßgerät eine Speichereinheit zur Speicherung von Referenzdaten auf. Mit Hilfe der Referenzdaten können die Korrekturwerte für das Drucksignal an den jeweiligen Benutzer angepaßt werden. Beispielsweise können für Benutzer unterschiedlicher Körpergröße verschiedene Referenzdatensätze abgespeichert sein.

Die Druckmessung kann grundsätzlich an verschiedenen Körpergliedern, beispielsweise an einem Fingern, vorgenommen werden. Vorzugsweise jedoch ist die Anlegeeinheit zum Anlegen des Drucksensors an ein Handgelenk ausgebildet. Die Druckerfassung am Handgelenk erlaubt bei einer einfach Erfassung der Ausrichtung eine hohe Meßgenauigkeit.

Nachfolgend wird die vorliegende Erfindung anhand von Ausführungsbeispielen und zugehöriger Zeichnungen näher erläutert. In diesen zeigen:
- Figur 1: ein Blutdruckmeßgerät entsprechend einer bevorzugten Ausführung der Erfindung, das an einem Handgelenk einer Person angelegt ist, in einer schematischen Darstellung,
- Figur 2: den Aufbau eines Blutdruckmeßgerätes entsprechend einer bevorzugten Ausführung der Erfindung in einer schematischen Darstellung,
- Figur 3: ein Flußdiagramm, das die einzelnen Schritte eines Verfahrens zur Bestimmung des Blutdrucks gemäß einer bevorzugten Ausführung der Erfindung zeigt, daß mit der Vorrichtung gemäß Figur 2 ausführbar ist,
- Figur 4: den Aufbau eines Blutdruckmeßgerätes gemäß einer weiteren Ausführung der Erfindung in einer Darstellung ähnlich Figur 2, und
- Figur 5: ein Flußdiagramm ähnlich Figur 3, das die Schritte eines Verfahrens zur Bestimmung des Blutdrucks entsprechend einer weiteren Ausführung der Erfindung zeigt, das mit Hilfe des Blutdruckmeßgerätes gemäß Figur 4 ausführbar ist.

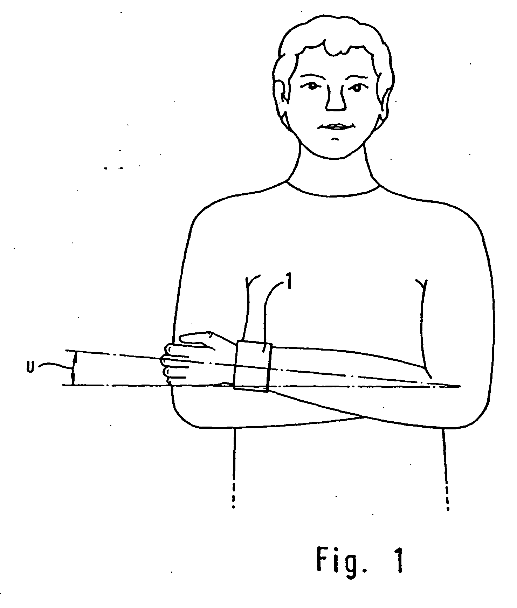

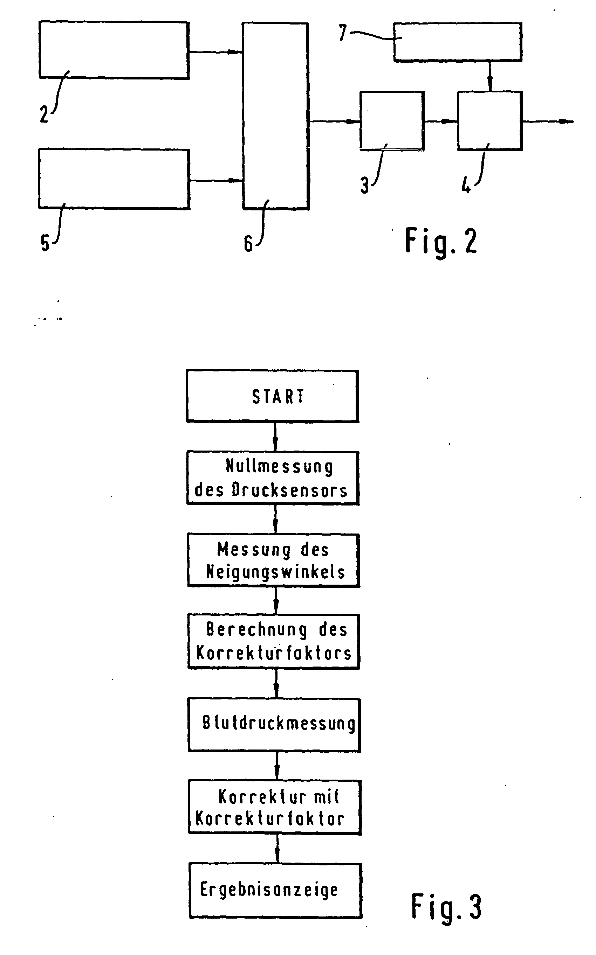

Das in Figur 1 gezeigte Blutdruckmeßgerät ist zur Messung des Blutdrucks am Handgelenk ausgebildet. Es weist als Anlegeeinheit eine Manschette 1 auf, mit der ein Drucksensor zur Signalaufnahme an die Innenseite des linken Handgelenkes angelegt werden kann. Die Manschette 1 weist eine Blase auf, die vorzugsweise mit Luft aufgepumpt wird, um während des Luftablassens mittels der ozillometrischen Messmethode den diastolischen, den systolischen, eventuell den mittleren Blutdruck- und den Puls zu bestimmen. Der Drucksensor 2 (Figur 2) kann z.B. als kapazitiver oder piezoresistiver Sensor ausgebildet sein.

Der Drucksensor 2 ist über einen Verstärker und Analog/Digital-Wandler 3 mit einer Auswerteeinheit 4 verbunden, die als Microcontroller ausgebildet und zur algorithmischen Auswertung des elektrischen Signales des Drucksensors ausgebildet ist.

Das Blutdruckmeßgerät weist ferner einen Neigungssensor 5 auf, der ebenfalls über den Verstärker und Analog/Digital-Wandler 3 mit der Auswerteeinheit 4 verbunden ist. Der Neigungssensor 5 erfaßt die Neigung der Manschette 1 und damit des Handgelenks bzw. des Unterarmes gegenüber der Horizontalen, d. h. der Neigungssensor 5 stellt ein elektrisches Signal bereit, das dem Neigungswinkel u des Handgelenks zur Horizontalen entspricht (vgl. Figur 1). Der Neigungssensor weist vorzugsweise ein beweglich gelagertes Teil, wie z.B. ein Pendel auf und ist mit einer Einrichtung versehen, mit der der Neigungswinkel u, die Geschwindigkeit oder die Beschleunigung des beweglichen Teils elektrisch erfaßbar ist. Je nach dem für welchen Zweck das detektierte Signal verwendet werden soll, um z.B. verschiedene Arten von Bewegungsartifakte zu unterscheiden, wird auf dem Neigungswinkel, die Geschwindigkeit oder die Beschleunigung des beweglichen Teils zurückgegriffen. Die Geschwindigkeit und die Beschleunigung des beweglichen Teils im Neigungssensor kann z.B. durch die elektronische Erzeugung der ersten und zweiten Ableitung oder durch speziell dafür ausgebildete Sensoren detektiert werden, um z.B. verschiedene Arten von Bewegungsartefakten zu unterscheiden und entsprechend zu berücksichtigen.

Wie Figur 2 zeigt, sind der Drucksensor 2 und der Neigungssensor 5 mit dem gemeinsamen Verstärker und Analog/Digital-Wandler 3 über ein Zeitschaltglied 6, das als Multiplexer ausgebildet ist, verbunden. Durch den Multiplexer 6 wird wahlweise das Signal des Drucksensors 2 oder das Signal des Neigungssensors 5 auf den Verstärker und Analog/Digital-Wandler 3 durchgeschaltet. Die in Figur 2 skizzierte Schaltung zur Signalaufbereitung ist vorzugsweise in einem Schaltkreis integriert, der abhängig von der Schaltstellung des Multiplexers 6 auch die Konfiguration des Verstärkers und Analog/Digital-Wandlers 3 den vom Sensor herrührenden Anforderungen anpaßt.

Um das Blutdruckmeßgerät an verschiedene Randbedingungen, insbesondere verschiedene Benutzer anpassen zu können, ist ein Referenzwertspeicher 7 vorgesehen, der mit der Auswerteeinheit 4 verbunden oder in ihr enthalten ist. Die individuellen Randbedingungen der Messung, die die Positionierung des Blutdruckmeßgerätes und damit das Meßergebnis beeinflussen, wie beispielsweise die Armlänge oder die Lage des Herzens, sind benutzerspezifisch verschieden. Es wird deshalb einmal eine Referenzmessung auf Herzniveau durchgeführt, deren Ergebnisse, insbesondere der dabei erfaßte Neigungswinkel uref und die von diesem Winkel abhängige Signalspannung, zusammen mit eventuellen Kalibrierdaten des Neigungssensors 5 im Referenzspeicher 7 abgelegt werden und bei allen nachfolgenden Blutdruckmessungen zur Korrektur dienen. Die Korrektur des jeweils gemessenen Blutdrucks ergibt sich dabei aus dem gemessenen Neigungswinkel u wie folgt:

Pkorr = Pₘₑₛₛ - k (1 - sin u/sin u_{ref}), wobei Pₖₒᵣᵣ und Pₘₑₛₛ die korrigierten bzw. gemessenen Druckwerte, u den gegenüber der Horizontallage des Blutdruckmeßgerätes gemessenen Winkel und u_{ref} den einmalig, gleichfalls gegen die Horizontallage bestimmten Referenzwinkel bei einer Lage des Geräts auf Herzniveau darstellen. Der Multiplikator k ist ein für die Kalibration erforderlicher konstanter Koeffizient. Entsprechend dieser Beziehung kann der tatsächlich gemessene Druck um den hydrostatischen Differenzdruck korrigiert und der auf der Herzhöhe zugehörige Blutdruck bestimmt werden. Ein lagebedingter systematischer Meßfehler wird dadurch vermieden.

Nachfolgend wird der Ablauf der Verfahrensschritte bei einer Blutdruckbestimmung in Verbindung mit Figur 3 näher erläutert. Zunächst wird vor der eigentlichen Messung eine Nullmessung des Drucksensors durchgeführt, um das Signalniveau des Drucksensors bei fehlendem Druck jedes Mal neu festzulegen und die Genauigkeit der Messung zu erhöhen. Sobald das Blutdruckmeßgerät mittels einer Manschette 1 am linken Handgelenk des Benutzers angelegt ist, bringt der Benutzer zur Blutdruckmessung die Arme in eine im wesentlichen vor dem Oberkörper verschränkte Position und seinen Oberkörper in eine im wesentlichen aufrechte Stellung (vgl. Figur 1). Dies stellt sicher, daß der erfaßte Neigungswinkel u in Korrelation steht mit der Lage des Handgelenkes relativ zum Herzen, d. h. daß der Neigungswinkel u ein Maß, für die Höhendifferenz zwischen der Lage des Handgelenkes und der Lage des Herzens ist. Um eine korrekte Meßstellung zu gewährleisten, ist vorzugsweise eine Anzeigevorrichtung des Blutdruckmeßgerätes derart angeordnet, daß diese vom Benutzer nur in einer korrekten Stellung ablesbar ist. Insbesondere kann die Anzeigevorrichtung an der oberen Schmalseite der Manschette 1, d. h. in dem Bereich, der im angelegten Zustand an der oberen Schmalseite des Gelenkes etwa in Fortsetzung des Daumengliedes liegt, angeordnet sein.

Hat der Benutzer die entsprechende Stellung eingenommen, wird zunächst mit dem Neigungssensor 5 der Neigungswinkel u des Blutdruckmeßgerätes bestimmt. Das Zeitschaltglied 6 schaltet das Signal des Neigungssensors 5 zu dem Verstärker und Analog/DigitalWandler 3 durch, so daß dieses von der Auswerteeinheit 4 ausgewertet werden kann. Dabei wird in einem nächsten Verfahrensschritt der Korrekturfaktor bestimmt, um den der tatsächlich gemessene Blutdruck dann zu korrigieren ist. Der im Handgelenk herrschende Blutdruck wird dann in einem weiteren Verfahrensschritt bestimmt. Hierzu wird das Signal des Drucksensors 2 von dem Zeitschaltglied 6 zur Auswerteeinheit durchgeschaltet. Anschließend wird der tatsächlich erfaßte Blutdruck um den zuvor bestimmten Korrekturwert korrigiert. Das korrigierte Meßergebnis des Blutdrucks wird dann an der Anzeigevorrichtung des Blutdruckmeßgerätes angezeigt.

Alternativ kann die Vermeidung lagebedingter Fehlmessungen auch über die Anzeigevorrichtung des Blutdruckmeßgerätes interaktiv mit dem Benutzer gesteuert werden. Hierzu erhält der Anwender solange Anzeichen auf der Anzeigevorrichtung des Blutdruckmeßgerät bis er es innerhalb eines vorgegebenen Winkeltoleranzfeldes um den Referenzwinkel gebracht hat und damit eine Meßposition eingenommen hat, in der keine Korrektur des Blutdruckmeßwertes mehr erforderlich ist, oder eine Korrektur durchgeführt werden muß. In einer weiteren Ausführungsform wird das Anzeichen auf der Anzeigevorrichtung zur Benutzerführung für die optimale Meßlage am Anfang und/oder während der Druckmessung eingesetzt. Beispielsweise weist die Anzeigevorrichtung als Anzeichen einen nach oben und nach unten weisenden Pfeil auf, von denen nur der gerade angezeigt wird oder blinkt, wenn der Benutzer sein Handgelenk mit dem Blutdruckmeßgerät entsprechend nach oben oder unten bewegen soll, um in die korrekte Meßposition zu gelangen. Eine Benutzerführung durch eine rote Lampe für eine schlechte Meßposition und / oder eine grün aufleuchtende Lampe für eine korrekte Meßposition oder auch durch ein akustisches Warnsignal bei einer schlechten Meßposition ist alternativ ebenso als Anzeichen ausbildbar. Vorzugsweise wird das Anzeigesignal für die korrekte/unkorrekte Meßposition nur vor der Messung an der elektronischen Anzeigevorrichtung (z.B. LCD) angezeigt. Alternativ können auch andere Zeitpunkte (z.B. auch während der Messung, z.B. für den Fall einer Veränderung der Meßposition) zur Anzeige programmiert werden. Dadurch, daß der Neigungssensor ein elektrisches Signal abgibt, ist es ohne weiteres möglich, abweichend von einer ständigen evtl. den Benutzer irritierenden Anzeige der Meßposition diese nur zu bestimmten Zeitpunkten elektronisch anzuzeigen.

Umgekehrt wird in einer weiteren Ausführungsform in einem Meßwertspeicher eine konkrete Validierung der Meßergebnisse hinsichtlich der detektierten Meßlage durchgeführt. Somit wird beispielsweise nach der Messung angezeigt, ob das Meßergebnis wegen einer ungünstigen Meßlage oder einer Bewegung während der Messung verworfen werden sollte oder ob eine Kompensierung bzw. Korrektur bzw. Eliminierung von lagebedingt fehlerhaften Meßwerten erfolgt ist.

Die in den obigen beiden Absätzen dargestellten Ausführungsformen sind sowohl in Kombination mit der ersten als auch mit der nachfolgend beschriebenen zweiten Ausführungsform kombinierbar.

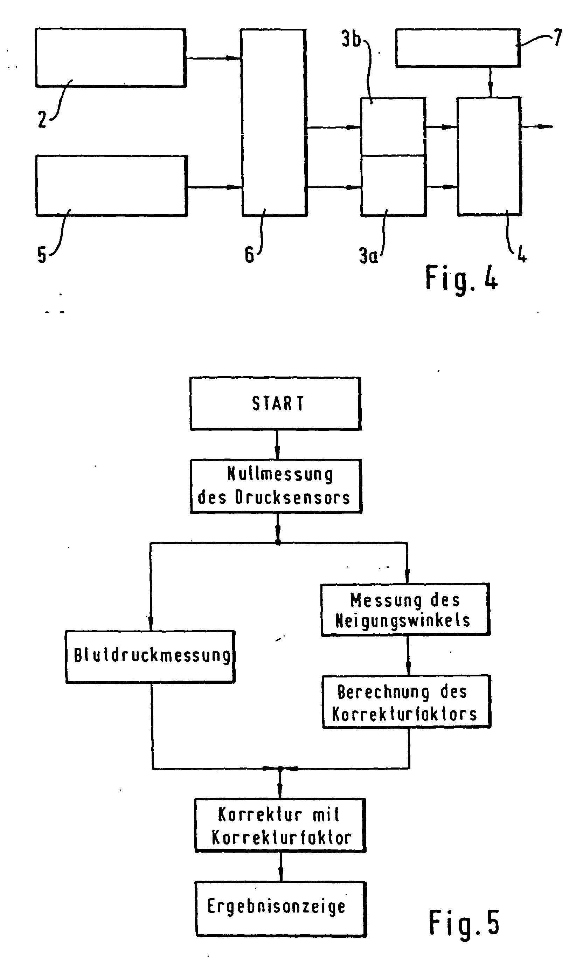

Eine zweite Ausführung eines erfindungsgemäßen Blutdruckmeßgerätes ist in Figur 4 dargestellt. Der Aufbau des Blutdruckmeßgerätes ist grundsätzlich ähnlich dem in Figur 2 gezeigten, so daß für dieselben Bauteile gleiche Bezugszeichen verwendet sind und eine nochmalige Beschreibung derselben Bauteile nicht notwendig ist. Die Ausführung gemäß Figur 4 unterscheidet sich im wesentlichen dadurch, daß der Drucksensor 2 und der Neigungssensor 5 nicht über einen gemeinsamen Verstärker und Analog/Digital-Wandler mit der Auswerteeinheit 4 verbunden sind, sondern daß sowohl der Neigungssensor 5 als auch der Drucksensor 2 jeweils separat über einen eigenen Verstärker und Analog/Digital-Wandler 3 a bzw. 3 b mit der Auswerteeinheit 4 verbunden sind. Hierdurch kann das Signal des Neigungssensors 5 kontinuierlich der Auswerteeinheit 4 bereitgestellt werden. Der Neigungswinkel u wird zeitparallel zur Erfassung des Blutdrucks im Handgelenk erfaßt.

Zur Erfassung einer Bewegung bzw. Beschleunigung des Handgelenkes weist die Auswerteeinheit 4 eine Differenziereinheit auf, mit Hilfe derer die zeitliche Ableitung des Signals des Neigungssensors 5, die dann der Bewegung bzw. (Winkel-) Geschwindigkeit entspricht, und die Ableitung der zeitlichen Ableitung, die dann der (Winkel-) Beschleunigung entspricht, bestimmbar ist.

Der Ablauf der Verfahrensschritte des Verfahrens zur Blutdruckbestimmung, das mit der Vorrichtung gemäß Figur 4 ausführbar ist, ist in Figur 5 dargestellt. Wie in Figur 5 zu sehen ist, wird der Neigungswinkel zeitparallel zur Blutdruckmessung bestimmt und zur Berechnung des Korrekturfaktors verwendet.

Aus der bestimmten Bewegung (Geschwindigkeit) bzw. Beschleunigung des Handgelenkes wird zunächst festgestellt, ob sich der Anwender generell in einem Zustand ausreichender Ruhe befindet und eine Messung sinnvoll durchführbar ist. Wird ein erhöhtes Maß an motorischer Aktivität festgestellt, so daß nur ein unrepräsentatives Blutdruckmeßergebnis geliefert werden kann, wird die Messung unterbunden bzw. auf der Anzeigevorrichtung ein Hinweis auf die geringe Aussagekraft der Messung angezeigt. Diese Ausbildung des Blutdruckmeßgerätes und dieses Verfahren der Anzeige aufgrund geringer Aussagekraft der Messung in Folge einer ungeeigneten Winkelstellung des Körpergliedes, an dem das Blutdruckmeßgerät befestigt ist oder aufgrund von bestimmten detektierten Bewegungen, die das Meßergebnis stark verfälschen würden, ist unabhängig von den übrigen Korrektur- und Anzeigemöglichkeiten oder in beliebiger Kombination durchführbar. Die Unterbindung der Messung bzw. der Hinweis auf geringe Aussagefähigkeit der Messung kann vor und oder während der Messung oder nach der Messung durchgeführt werden.

Darüber hinaus wird der gemessene Blutdruck mit Hilfe der erfaßten Bewegung (Geschwindigkeit) und Beschleunigung korrigiert. Kurzzeitige Lageänderungen wie beispielsweise Kurzzeitbewegungen oder Zittern des Handgelenkes während der Blutdruckmessung machen sich in erster Linie durch Schwankungen im Ausgangssignal des Drucksensors bemerkbar. Durch eine Rückrechnung von den gemessenen Lageschwankungen auf die dazu korrespondierende Druckschwankung kann der Einfluß der Bewegungsartefakte verringert werden.

Das durch den Neigungssensor 5 erzeugte Meßsignal wird vorzugsweise elektronisch nachgearbeitet, so daß ein gedämpftes schwingungsverhaltende des beweglichen Teils im Neigungssensor mit kleineren schwingungsamplituden und einem kürzeren Ausschwingen als mechanisch generiert elektronisch durch Filterung, Integration, bzw. Mittelung über die Periodendauer des beweglichen Teils im Drucksensor erzeugt wird. Gegenüber mechanischen Dämpfungssystemen ist dadurch ein optimales Dämpfungsverhalten (auch ein nicht lineares) unabhängig von der Fertigungsqualität des beweglichen Teils im Drucksensor einstellbar. Dies ermöglicht auch eine wesentlich besserere Ablesbarkeit des gemessenen Signals an der Anzeigevorrichtung.

Die Anzeigevorrichtung auf der das aufgebereitete Meßergebnis des Neigungssensors angezeigt wird ist dieselbe, mit der die Blutdruckmeßwerte und gegebenenfalls der Puls angezeigt wird. Es handelt sich dabei um eine elektronische Anzeigeeinrichtung vom Typ z.B. eines LCD, ST, STN, TFT oder ähnlichen Displays. Es sind grundsätzlich z.B. alle Displaytypen verwendbar, die auch für tragbare Computeranzeigevorrichtungen eingesetzt werden. Nachdem bekanntermaßen die Ablesbarkeit von Anzeigen auf diesen Anzeigevorrichtungen vom Betrachtungswinkel relativ zur Anzeigevorrichtung abhängt, kann in einer weiteren Ausführungsform das Meßergebnis des Neigungssensors ergänzend oder ausschließlich dafür verwendet werden, daß in einer Anpassungseinrichtung die elektronische Anzeigeeinrichtung an den Blickwinkel angepaßt wird, da in der Regel ein Zusammenhang zwischen den möglichen Neigungswinkeln am Handgelenk und den möglichen Blickwinkeln relativ zum Blutdruckmeßgerät mit Anzeigevorrichtung am Handgelenk besteht.

Das Ergebnis der beschriebenen Nullmessung des Drucksensors 2 kann auch in dem Referenzwertspeicher 7 abgelegt werden, so daß je Bedienperson nur insgesamt einmal eine spezifische Kalibrierung stattfinden muß und später nur noch die Bedienperson einzugeben ist.

Unter dem Begriff Drucksensor im Sinne der vorliegenden Anmeldung sind allgemein Sensoren zu verstehen, die ein Drucksignal bereitstellen, aus dem sich der in dem Körperglied herrschende Blutdruck bestimmen läßt. Dies kann beispielsweise auch ein optischer Sensor, wie beispielsweise ein Infrarotsensor sein, der den Puls optisch erfassen kann, so daß sich aus der zeitlichen Veränderung des Signales der Manschetteninnendruck detektieren läßt.

## Patentansprüche

1. Verfahren zur Bestimmung des Blutdrucks, wobei eine Manschette vorgesehen ist, mit der ein Drucksensor und eine Anzeigevorrichtung zur Anzeige der Meßergebnisse am Handgelenk angelegt wird, wobei ein in dem Handgelenk herrschender Blutdruck mittels der oszillometrischen Meßmethode und die Ausrichtung des Handgelenks mit einem Neigungssensor innerhalb eines Blutdruckmeßgerätegehäuses erfaßt wird, wobei der Neigungssensor ein elektrisches Signal je nach der detektierten Ausrichtung des Handgelenkes abgibt und dieses elektrische Signal weiterverarbeitet wird und wobei das Handgelenk zur Blutdruckmessung in eine Position anliegend vor dem Oberkörper angeordnet wird, so daß das elektrische Signal ein Maß für die Lage des Handgelenks relativ zum Herzen ist.

2. Verfahren nach Anspruch 1, wobei die Anzeigevorrichtung derart angeordnet wird, daß diese nur in einer korrekten Stellung vor dem Oberkörper ablesbar ist.

3. Verfahren nach einem der vorhergenden Ansprüche, wobei die Anzeigevorrichtung an der oberen Schmalseite des Handgelenks etwa in Fortsetzung des Daumengliedes angeordnet wird.

4. Verfahren nach Anspruch 1, wobei die Winkelstellung (u) des Handgelenks, bzw. des Unterarmes, mit dem Neigungssensor (5) erfaßt wird.

5. Verfahren nach einem der vorhergenden Ansprüche, wobei eine Bewegung, insbesondere Geschwindigkeit oder Beschleunigung, des Handgelenks während der Druckerfassung erfaßt wird, so daß festgestellt wird, ob sich das Handgelenk in ausreichender Ruhe befindet.

6. Verfahren nach zumindest einem der vorhergehenden Ansprüche, wobei über eine Anzeigevorrichtung anwenderinteraktiv ein Anzeichen eine Rückmeldung ausgibt, ob die Meßposition in einem korrekten Winkelbereich des Handgelenkes an dem die Messung erfolgt liegt.

7. Verfahren nach Anspruch 6, wobei das Anzeichen den Anwender interaktiv zur korrekten Meßposition führt.

8. Blutdruckmeßgerät mit einem Drucksensor zur Erzeugung eines Drucksignals, einer Manschette zum Anlegen des Drucksensors an ein Handgelenk und einer Auswerteeinheit zur Auswertung des Drucksignals, wobei ein Neigungssensor (5), der innerhalb eines Blutdruckmeßgerätegehäuses angeordnet ist, zur Erfassung der Ausrichtung des Handgelenks vorgesehen ist, wobei durch den Neigungssensor (5) zur Weiterverarbeitung ein elektrisches Signal in Abhängigkeit von der Ausrichtung des Handgelenks abgebbar ist und wobei die Anzeigevorrichtung derart angeordnet ist, daß diese nur in einer korrekten Stellung, bei der das Blutdruckmeßgerät vor dem Oberkörper anliegend angeordnet ist, ablesbar ist.

9. Blutdruckmeßgerät nach Anspruch 8, wobei das Blutdruckmeßgerät derart ausgebildet ist, daß die Anzeigevorrichtung an der oberen Schmalseite des Handgelenks etwa in Fortsetzung des Daumengliedes angeordnet ist.

10. Blutdruckmeßgerät nach Anspruch 8 oder 9, wobei der Neigungssensor (5) die Neigung des Handgelenks, an dem der Drucksensor (2) angelegt ist, erfaßt.

11. Blutdruckmeßgerät nach Anspruch 8, 9 oder 10, wobei eine Bewegungs-Erfassungseinheit (5) zur Erfassung einer Bewegung, insbesondere einer Geschwindigkeit oder Beschleunigung, des Handgelenks vorgesehen ist.

12. Blutdruckmeßgerät nach Anspruch 11, wobei die Bewegungs-Erfassungseinheit den Neigungssensor (5) und eine mit diesem verbundene Differenziereinheit umfaßt.

13. Blutdruckmeßgerät nach zumindest einem der Ansprüche 8 bis 12, wobei der Neigungssensor (5) und der Drucksensor (2) mit der Auswerteeinheit (4) über eine Zeitschalteinheit (6) verbunden sind.

14. Blutdruckmeßgerät nach einem der vorhergehenden Ansprüche 8 bis 13, wobei die Ausrichtung des Handgelenks zeitparallel zur Blutdruckmessung erfaßbar ist.

15. Blutdruckmeßgerät nach einem der Ansprüche 8 bis 14, wobei die Messung unterbindbar ist oder ein Hinweis auf die geringe Aussagekraft der Messung an der Anzeigevorrichtung anzeigbar ist, sobald der Neigungssensor eine Stellung des Handgelenks erfaßt, die zu einem ungenauen Blutdruckmeßergebnis führt.

16. Blutdruckmeßgerät nach einem der Ansprüche 8 bis 15, wobei durch die Anzeigevorrichtung Anzeichen zur Benutzerführung in die optimale Meßlage anzeigbar sind.

17. Blutdruckmeßgerät nach Anspruch 16, wobei durch die Anzeichen ein korrekter und/oder unkorrekter Winkelbereich oder eine Bewegung des Blutdruckmeßgeräts und/oder ein Hinweis zur korrekteren Meßlage ausgebbar ist.

18. Blutdruckmeßgerät nach Anspruch 16 oder 17, wobei als Anzeichen zur Benutzerführung Richtungspfeile vorgesehen sind, die entsprechend der für die optimale Meßlage erforderlichen Position des Handgelenks anzeigbar sind.

19. Blutdruckmeßgerät nach einem der Ansprüche 8 bis 18, wobei ein Meßwertspeicher und / oder eine Einrichtung vorgesehen ist, zur Validierungsbestimmung der Meßergebnisse, so daß abhängig von der Meßlage, dem Meßneigungswinkel, oder der Bewegung während der Messung ein Hinweis über die fehlerhafte Meßbedingungen ausgebbar ist.

## Claims

1. Method for determining the blood pressure, wherein a cuff is provided with which a pressure sensor and a display device for displaying the measurement results are applied to a person's wrist, a blood pressure prevailing in the wrist being detected by means of the oscillometric measurement method, and the alignment of the wrist being detected by a gradient sensor inside the blood pressure monitor housing; the gradient sensor delivering an electric signal depending on the detected alignment of the user's wrist and this electric signal being processed further; and the wrist being arranged in a position in contact with and in front of the upper body for the purpose of the blood pressure measurement, such that the electric signal is a measure of the position of the wrist in relation to the heart.

2. Method according to Claim 1, wherein the display device is arranged so that it can be read only in a correct position in front of the body.

3. Method according to any one of the preceding claims, wherein the display device is arranged on the upper narrow side of the wrist in approximate continuation of the thumb.

4. Method according to Claim 1, wherein the angular position (u) of the wrist and/or the forearm is detected by the gradient sensor (5).

5. Method according to any one of the preceding claims, wherein a movement, in particular a speed or acceleration of the wrist, is detected during the pressure measurement, thus making it possible to ascertain whether the wrist is motionless enough for a measurement.

6. Method according to at least one of the preceding claims, wherein an indicator outputs feedback via a display device regarding whether the measurement position is in a correct angular range of the wrist on which the measurement is performed and does so in a user-interactive manner.

7. Method according to Claim 6, wherein the indicator leads the user interactively to the correct measurement position.

8. Blood pressure monitor having a pressure sensor for generating a pressure signal, a cuff for applying a pressure sensor to a person's wrist and an analyzer unit for analyzing the pressure signal, wherein a gradient sensor (5) which is arranged inside a blood pressure monitor housing is provided for detecting the alignment of the wrist, an electric signal being deliverable by the gradient sensor (5) as a function of the alignment of the wrist for further processing, and the display device being arranged so that it can be read only in the correct position in which the blood pressure monitor is situated in front of and in contact with the upper body.

9. Blood pressure monitor according to Claim 8, wherein the blood pressure monitor is designed so that the display device is arranged on the upper narrow side of the wrist approximately in continuation of the thumb.

10. Blood pressure monitor according to Claim 8 or 9, wherein the gradient sensor (5) detects the inclination of the wrist on which the pressure sensor (2) is attached.

11. Blood pressure monitor according to Claim 8, 9 or 10, wherein a movement detection unit (5) is provided for detecting a movement in particular a speed or acceleration of the wrist.

12. Blood pressure monitor according to Claim 11, wherein the movement detection unit includes the gradient sensor (5) and a differentiating unit connected thereto.

13. Blood pressure monitor according to at least one of Claims 8 through 12, wherein the gradient sensor (5) and the pressure sensor (2) are connected to the analyzer unit (4) via a timing unit (6).

14. Blood pressure monitor according to any one of the preceding Claims 8 through 13, wherein the alignment of the person's wrist can be detected in parallel with the blood pressure measurement.

15. Blood pressure monitor according to any one of Claims 8 through 14, wherein the measurement can be suppressed or indication of the low informational value of the measurement can be displayed on the display device as soon as the gradient sensor detects a position of the wrist which would lead to an inaccurate blood pressure measurement result.

16. Blood pressure monitor according to any one of Claims 8 through 15, wherein signs for user guidance to the optimal measurement position can be displayed by the display device.

17. Blood pressure monitor according to Claim 16, wherein a correct and/or incorrect angular range or a movement of the blood pressure monitor and/or a notice about a more correct measurement position can be output through these signs.

18. Blood pressure monitor according to Claim 16 or 17, wherein directional arrows are provided as signs for user guidance, these arrows being displayable according to the required position of the person's wrist for the optimal measurement position.

19. Blood pressure monitor according to any one of Claims 8 through 18, wherein a measured value memory and/or a device are provided for validation determination of the measurement results so that depending on the measurement position, the measurement angle of inclination or movement during the measurement, a message about the defective measurement conditions can be output.

## Revendications

1. Procédé pour déterminer la tension artérielle, une manchette étant prévue permettant de poser sur le poignet un capteur de pression et un dispositif d'affichage pour afficher les résultats de mesure, une tension artérielle régnant dans le poignet étant détectée au moyen de la méthode de mesure oscillométrique et l'orientation du poignet étant détectée avec un capteur d'inclinaison à l'intérieur d'un boîtier de tensiomètre, le capteur d'inclinaison délivrant un signal électrique, en fonction de l'orientation détectée du poignet et ledit signal électrique étant traité ultérieurement et le poignet étant placé pour la mesure de la tension artérielle dans une position en appui contre le devant du torse, pour que le signal électrique soit une mesure de la position du poignet par rapport au coeur.

2. Procédé selon la revendication 1, le dispositif d'affichage étant disposé de sorte que ce dernier ne soit lisible que dans une position correcte, à l'avant du torse.

3. Procédé selon l'une quelconque des revendications précédentes, le dispositif d'affichage étant disposé sur le côté étroit supérieur du poignet, environ dans le prolongement de la phalange du pouce.

4. Procédé selon la revendication 1, la position angulaire (u) du poignet ou de l'avant-bras étant détectée par le capteur d'inclinaison (5).

5. Procédé selon l'une quelconque des revendications précédentes, un déplacement, notamment une vitesse ou une accélération du poignet étant détecté pendant la détection de la tension, pour déterminer si le poignet est suffisamment au repos.

6. Procédé selon au moins l'une quelconque des revendications précédentes, par l'intermédiaire d'un dispositif d'affichage, de façon interactive avec l'utilisateur, un indice donnant une information en retour, si la position de mesure se situe dans une plage angulaire correcte du poignet sur lequel a lieu la mesure.

7. Procédé selon la revendication 6, l'indice conduisant l'utilisateur de façon interactive à la position de mesure correcte.

8. Tensiomètre avec un capteur de pression pour la génération d'un signal de pression, une manchette pour l'application du capteur de pression sur un poignet et une unité d'évaluation, pour l'évaluation du signal de pression, un capteur d'inclinaison (5), qui est disposé à l'intérieur d'un boîtier de tensiomètre étant prévu pour la détection de l'orientation du poignet, le capteur d'inclinaison (5) étant susceptible de délivrer pour un traitement ultérieur un signal électrique en fonction de l'orientation du poignet et le dispositif d'affichage étant placé de sorte à n'être lisible que dans une position correcte, dans laquelle le tensiomètre est placé en appui contre le devant du torse.

9. Tensiomètre selon la revendication 8, le tensiomètre étant conçu de sorte que le dispositif d'affichage soit disposé sur le côté étroit supérieur du poignet, environ dans le prolongement de la phalange du pouce.

10. Tensiomètre selon la revendication 8 ou 9, le capteur d'inclinaison (5) détectant l'inclinaison du poignet sur lequel est posé le capteur de pression (2).

11. Tensiomètre selon la revendication 8, 9 ou 10, une unité de détection de déplacement (5) étant prévue pour la détection d'un déplacement, notamment d'une vitesse ou d'une accélération du poignet.

12. Tensiomètre selon la revendication 11, l'unité de détection de déplacement comprenant le capteur d'inclinaison (5) et une unité différentiatrice reliée à ce dernier.

13. Tensiomètre selon au moins l'une quelconque des revendications 8 à 12, le capteur d'inclinaison (5) et le capteur de pression (2) étant reliés avec l'unité d'évaluation (4) par l'intermédiaire d'une unité de minuterie (6).

14. Tensiomètre selon l'une quelconque des revendications précédentes 8 à 13, l'orientation du poignet étant détectable en parallèle dans le temps avec la mesure de la tension artérielle.

15. Tensiomètre selon l'une quelconque des revendications 8 à 14, la mesure pouvant être arrêtée, ou une information concernant la faible force probante de la mesure étant affichable sur le dispositif d'affichage, dès lors que le capteur d'inclinaison détecte une position du poignet qui conduit à un résuftat imprécis de la mesure de la tension artérielle.

16. Tensiomètre selon l'une quelconque des revendications 8 à 15, le dispositif d'affichage étant susceptible d'afficher des indices pour guider l'utilisateur dans la position de mesure optimum.

17. Tensiomètre selon la revendication 16, les indices permettant d'éditer une plage angulaire correcte et/ou incorrecte ou un déplacement du tensiomètre et/ou une information concernant la position de mesure correcte.

18. Tensiomètre selon la revendication 16 ou 17, des flèches de direction, affichables en fonction de la position du poignet nécessaire pour la position de mesure optimum étant prévues comme indices pour guider l'utilisateur.

19. Tensiomètre selon l'une quelconque des revendications 8 à 18, une mémoire des valeurs mesurées et/ou un système étant prévus pour déterminer la validation des résultats mesurés, pour qu'en fonction de la position de mesure, de l'angle d'inclinaison de mesure ou du déplacement pendant la mesure, une information concernant les conditions de mesure erronées soit susceptible d'être éditée.
